(19) **Europäisches Patentamt**

**European Patent Office**

**Office européen des brevets**

(11) Veröffentlichungsnummer: **0 100 964**

**A2**

## (12) EUROPÄISCHE PATENTANMELDUNG

(21) Anmeldenummer: **83107394.5**

(22) Anmeldetag: **27.07.83**

(51) Int. Cl.³: **A 61 K 9/50**

(30) Priorität: 31.07.82 DE 3228629
03.05.83 DE 3316012

(43) Veröffentlichungstag der Anmeldung:
22.02.84 Patentblatt 84/8

(84) Benannte Vertragsstaaten:
AT BE CH DE FR GB IT LI LU NL SE

(71) Anmelder: **Reifenrath, Rainer, Dr.**
**Berliner Strasse 42**
**D-3104 Unterlüss(DE)**

(72) Erfinder: **Reifenrath, Rainer, Dr. med.**
**Berliner Strasse 42**
**D-3104 Unterlüss(DE)**

(72) Erfinder: **Werner, Rolf-Günter, Dr. Dipl.-Mikrobiologe**
**Beim Fohrhäldele 18**
**D-7950 Biberach 1(DE)**

(74) Vertreter: **Sommer, Fritz, Dr. et al,**
**DR. KARL THOMAE GMBH Patentstelle Postfach 1755**
**D-7950 Biberach/Riss(DE)**

(54) **Pharmazeutisches Erzeugnis zur Behandlung und Prophylaxe von Infektionen sowie von Husten und obstruktiven Atemwegserkrankungen sowie Verfahren zu seiner Herstellung.**

(57) Die Erfindung betrifft ein pharmazeutisches Erzeugnis zur Behandlung und Prophylaxe von Infektionen, vor allem bakterieller, viraler oder Pilz-Infektionen, das auch - für den Respirationstrakt - als Antitussivum und Anti-Obstruktivum wirksam ist. Weiterhin betrifft die Erfindung ein Verfahren zur Herstellung dieses pharmazeutischen Erzeugnisses.

Der Erfindung hat die Aufgabe zugrunde gelegen, ein unspezifisch wirkendes Medikament gegen Infektionen, Husten oder obstruktive Atemwegserkrankungen zu schaffen, bei dem die schädlichen Nebenwirkungen der für diese Zwecke bekannten Präparate nicht auftreten und für das auch die Gegenindikationen der bekannten Präparate nicht gegeben sind.

Gegenstand der Erfindung ist ein pharmazeutisches Erzeugnis zur Behandlung und Prophylaxe von Infektionen, Husten und obstruktiven Atemwegserkrankungen, das aus Lecithin und/oder Cholesterin-Partikeln besteht.

Das Herstellungsverfahren gemäß der Erfindung besteht darin, daß Lecithin und/oder Cholesterin in der durch die gewünschte Zusammensetzung vorgegebenen Gewichtsmenge in Chloroform gelöst werden, die so entstandene Lipidlösung mit Wasser überschichtet und anschließend kontinuierlich gemischt wird bis zu ihrer Umwandlung in flüssigen Schaum, der dann bis zur vollständigen Verdunstung des Chloroforms mit Ultraschall behandelt wird.

Croydon Printing Company Ltd.

0100964

Dr. med. Rainer Reifenrath
Berliner Straße 42
D-3104 Unterlüß

Case 11/007
Dr.Fl./Kp

Pharmazeutisches Erzeugnis zur Behandlung und Prophylaxe
von Infektionen sowie von Husten und obstruktiven Atemwegserkrankungen sowie Verfahren zu seiner Herstellung

Die Erfindung betrifft ein pharmazeutisches Erzeugnis zur
Behandlung und Prophylaxe von Infektionen, vor allem bakterieller, viraler und Pilz-Infektionen, das auch - für den
Respirationstrakt - als Antitussivum und Anti-Obstruktivum
wirksam ist. Weiterhin betrifft die Erfindung ein Verfahren
zur Herstellung dieses pharmazeutischen Erzeugnisses.

Die bekannten Medikamente zur Behandlung von Infektionen
lassen sich in zwei Gruppen unterteilen, nämlich in:

1. Antimikrobiell wirkende Mittel (Antibiotika, Sulfonamide,
Antimykotika, Virostatika).
Diese Medikamente greifen die Infektionserreger direkt an.
Die mit ihnen bewirkte Therapie zielt auf eine Unschädlichmachung der Infektionserreger durch deren Vernichtung oder
Vermehrungsbehinderung ab. Die bekannten Medikamente dieser
Art haben folgende therapeutische Nachteile bzw. Unzulänglichkeiten:
- Ihre Wirkung ist spezifisch, d.h. ein bestimmtes Medikament wirkt nur gegen jeweils einen Teil des Erregerspektrums. Gegen Viren sind die bekannten Antibiotika nicht
wirksam.

- Es kann bei den Infektionserregern zur Resistenz-Entwicklungen durch Selektion der Erreger kommen, mit der Folge, daß resistente Erregerstämme heraus gezüchtet werden, gegen die das ursprünglich gegen die Erreger eingesetzte Medikament unwirksam ist.

- Es kann zu allergischen Reaktionen bis zum anaphylaktischen Schock kommen,

- Neben allergischen Reaktionen können passagere Nebenwirkungen auftreten, z.B. von Seiten des Magen-Darmtraktes, die zum Absetzen der Therapie zwingen. Insbesondere Antimykotika sind u.U. mit erheblichen Nebenwirkungen auf verschiedene Organe behaftet.

- Die meisten antimikrobiell wirkenden Mittel sind während des ersten Trimenons der Schwangerschaft kontraindiziert.

- Hochaktive antimikrobielle Chemotherapeutika sind sehr kostenaufwendig.

2. Die körpereigene Immunabwehr stimulierende Mittel (Impfstoffe).

Die mit diesen Medikamenten ausgeübte Therapie zielt auf eine Stimulierung der körpereigenen Immunabwehr gegen die Infektionserreger ab, sodaß die körpereigenen Abwehrstoffe die Antigene der Erreger unschädlich machen können. Außer der Schutzimpfung gibt es in den meisten Fällen keine gezielte antivirale Therapie mit Hilfe der bekannten Medikamente. Virostatika sind im wesentlichen extern anwendbar und haben sehr begrenzte Einsatzmöglichkeiten.

Die bekannten Antitussiva lassen sich in zwei Gruppen einordnen, nämlich in:

1. Die pflanzlichen Antitussiva

Diese bestehen entweder aus Efeublättertrockenextrakt als Einzelstoff oder aus Kombinationen von Destillaten aus Thymian, Anis, Fenchel, Salbei, Eucalyptus, Pfefferminz und Kamille. Die meisten gebräuchlichen Antitussiva enthalten Äthanol und sind daher bei Alkoholkranken kontraindiziert.

- 3 -

## 2. Die chemisch definierten Antitussiva

Diese lassen sich unterteilen in:

a) die Morphinderivate:
Sämtliche Morphinderivate können zu einer Beeinträchtigung des Reaktionsvermögens führen. Überdies kann eine Obstipation als Nebenwirkung auftreten. Die verwendeten Konservierungsstoffe können Hautreaktionen bewirken. Morphinderivate können nicht bei Säuglingen und stillenden Müttern eingesetzt werden. Außerdem sind Gewöhnung und Sucht häufig.

Gegenanzeigen sind alle Krankheitszustände, bei denen eine Dämpfung des Atemzentrums vermieden werden muß.

b) andere Antitussiva:
Diese beinhalten zum Teil Äthanol (bis zu 75 Volumen-%). Der größte Teil der bekannten Antitussiva aus dieser Gruppe beeinträchtigt das Reaktionsvermögen (z.B. Noscapin, Oxeladincitrat, Pipacetat-HCL, Levopropoxyphen-dibutinat, Dibenproperin-embonat u.a.).
Ein Teil dieser Präparate kann während des ersten Trimenons der Schwangerschaft nicht angewandt werden.

c) Kombinationen aus Morphinderivaten und anderen Stoffen:
Diese Kombinationen enthalten teilweise Äthanol, sie beeinträchtigen größtenteils das Reaktionsvermögen. Als Nebenwirkungen sind aufgetreten: Hautreaktionen, Mundtrockenheit, Miktionsbeschwerden und andere nachteilige Folgeerscheinungen. Gegenindikationen sind Krankheiten, bei denen eine Dämpfung des Atemzentrums zu vermeiden ist.

d) andere Kombinationen:

Andere Kombinationen von Antitussiva sind in der Roten Liste von 1982 unter den Nummern 20043 bis 20052 aufgeführt. Mit Ausnahme von zwei Präparten sind für diese Präparate die vorstehend erwähnten Nebenwirkungen genannt, teilweise andere Nebenwirkungen.

Es ist in diesem Zusammenhang zu erwähnen, daß 8 % aller Medikamenten-Intoxikationen durch Hustenmittel entstehen.

Die für inhalative Zwecke zur Verfügung stehenden Substanzen werden im wesentlichen als Sekretolytika bzw. Expectorantien benutzt. Spezifische Antitussiva sind für inhalative Zwecke nicht gebräuchlich. Nach dem Stande der Heilkunde sind Antitussiva somit keine unmittelbar und lokal an den Atemwegen angreifenden Substanzen, sondern sie werden als über den Magen-Darm-Trakt aufzunehmende Substanzen eingesetzt. Auch ist ihr Wirkungsmechanismus im allgemeinen kein lokaler, d.h. auf die Hustenrezeptoren in den Atemwegen wirkender, sondern überwiegend ein in entsprechenden Hirnarealen zentral-dämpfender.

Der Erfindung hat die Aufgabe zugrunde gelegen, ein unspezifisch wirkendes Medikament gegen Infektionen, Husten oder obstruktive Atemwegserkrankungen zu schaffen, bei dem die schädlichen Nebenwirkungen der für diese Zwecke bekannten Präparate nicht auftreten und für das auch die Gegenindikationen der bekannten Präparate nicht gegeben sind.

Gegenstand der Erfindung ist ein pharmazeutisches Erzeugnis zur Behandlung und Prophylaxe von Infektionen, Husten und obstruktiven Atemwegserkrankungen, das aus Lecithin und/oder Cholesterin-Partikeln besteht.

Das Herstellungsverfahren gemäß der Erfindung besteht darin, daß

a) Lecithin und/oder Cholesterin in der durch die gewünschte Zusammensetzung vorgebenen Gewichtsmenge in einem mit Wasser nicht mischbaren geeigneten Lösungsmittel wie Chloroform gelöst werden,

b) die so entstandene Lipidlösung mit Wasser überschichtet und anschließend kontinulierlich gemischt wird bis zu ihrer Umwandlung in flüssigen Schaum,

c) der dann bis zur vollständigen Verdunstung des Lösungsmittels mit Ultraschall behandelt wird und

gewünschtenfalls eine so erhaltene Lipidpartikelsuspension einem Trocknungsvorgang unterworfen wird.

Die Zusammensetzung der Lipidsuspension gemäß der Erfindung, d.h. das molare Gewichtsverhältnis der beiden Substanzen Lecithin und Cholesterin in der Suspension ist bestimmend für gewisse oberflächenaktive Eigenschaften des Erzeugnisses, insbesondere für die Kinetik der Bildung von grenzflächenaktiven Filmen. Die Lipide führen zu einer Inhibition der Adhäsion von Mikroorganismen an die Zell-Oberläche. Die physiko-chemischen Eigenschaften der Suspensionen gemäß der Erfindung bewirken auch einen antitussiven und antiobstruktiven Effekt.

Lecithin und Cholesterin sind beides körpereigne Substanzen. Die L-α-Dipalmitoyllecithin und Cholesterin sind beides natürliche Bestandteile biologischer Membranen, insbesondere auch der extracellulären Auskleidungsschicht der Atemwege. Sowohl elektronenmikroskopisch als auch biochemisch ist das Vorkommen von Lipid-Surfactant auch an anderen inneren Oberflächen und Grenzflächen, wie z.B. Conjunctiven, Pleura, Synovia, Pericard, Harnblase, eustachische Röhre, gesichert.

L-131

Das Gewichtsverhältnis der Substanzen Lecithin und Cholesterin, das für die anzuwendende Suspension gewählt wird, bestimmt sich nach therapeutischen Gesichtspunkten in Abhängigkeit von der ärztlichen Diagnose. Das Lecithin-Cholesterin-Gewichtsverhältnis dürfte in den meisten Fällen im Bereich von 1:2 bis 12:1, vorzugsweise jedoch von 2:1 bis 8:1, liegen.

Es sei angenommen, die Konzentration der Lipid-Partikel in der wässrigen Suspension solle 0,02 gr je 100 ml Wasser betragen bei einem Lecithin-Cholesterin-Gewichtsverhältnis von ca. 6:1. Dann müßten ca. 17,1 mg Lecithin und ca. 2,9 mg Cholesterin in 100 ml Wasser suspendiert werden.

Lecithin und Cholesterin sind als Lipide nicht wasserlöslich. Sie können in organischen Lösungsmitteln gelöst werden, doch scheiden diese Lösungsmittel wegen ihrer Toxizität für einen Transport der Lipide in den Organismus des Menschen aus. Es verbleibt daher nur die Möglichkeit, diese Lipide entweder ohne Transportmedium, also als Trockensubstanz, oder in wässriger Phase in den Organismus zu transportieren.

Das Verfahren nach der Erfindung benutzt Maßnahmen, um die Lipide Lecithin und Cholesterin in wässriger Phase zu suspendieren, um damit die Möglichkeit zu schaffen, diese Lipide in wirksamer Form mittels Inhalation, Instillation oder Injektion in den Organismus zu bringen.

Zu diesem Zweck werden die für die vorgesehene Konzentration und das gewünschte Gewichts-Mischungsverhältnis benötigten Gewichtsmengen Lecithin und Cholesterin in Chloroform gelöst, z.B. in 5 ml Chloroform. Dieser Lösung wird das für die gewünschte Konzentration benötigte Volumen an dreifach destilliertem Wasser durch Überschichten zugefügt. Die Chloroform- und Wasserphase werden sodann kontinuierlich gemischt, z.B. mittels eines Magnetrührers. Dabei entsteht ein flüssiger Schaum. Aus diesem Schaum wird durch Behandlung mit Ultraschall, z.B. mit einer Leistung von 90 bis 100 Watt, eine Emulsion hergestellt. Wird die Beschallung lange genug fortgesetzt, z.B. für die angegebene Chloroformmenge von 5 ml 5 Stunden lang, so verdunstet das Chloroform vollständig. Um die Beschallungszeit zu verkürzen, kann man mit höheren Konzentrationen der Lipid-Chloroform-Lösung arbeiten. Wenn man z.B. die angegebene Menge von 5 ml Chloroform auf 1 ml herabsetzt, so kann man dadurch die Beschallungszeit auf 30 Minuten verringern.

Da zum Überschichten der Lipid-Lösung in Chloroform destilliertes Wasser benutzt wird, kann durch Zusatz von NaCl zur fertigen Suspension eine Isotonie hergestellt werden.

Statt einer Mischung der Lipide Lecithin und Cholesterin kann zwecks Herstellung des Erzeugnisses nach der Erfindung auch nur Lecithin oder nur Cholesterin als Einzelstoff in wässriger Phase suspendiert werden.

Die nachfolgenden Beispiele sollen die Erfindung näher erläutern ohne diese zu beschränken:

Beispiel 1

0,001 mg Gesamtlipid pro ml Flüssigkeit

0,08 mg L-$\alpha$-Dipalmitoyl-lecithin und 0,02 mg Cholesterin werden in 5 ml Chloroform gelöst. Zu dieser Lösung werden 100 ml dreifach destilliertes Wasser hinzugefügt. Die Chloroform- und Wasserphase werden mittels Magnetrührer kontinuierlich gemischt, gleichzeitig wird das Gemisch durch Ultraschall mit einer Leistung zwischen 90-100 Watt behandelt. Die Beschallung der so entstehenden Emulsion wird solange fortgesetzt, bis das Chloroform vollständig verdunstet ist, d.h. bis eine Lipidsuspension in wässriger Phase entstanden ist. Dies dauert etwa 5 Stunden.

Zum Zumischen kann anstatt des Wassers auch eine Pufferlösung, z.B. ein Phosphatpuffer, benutzt werden.

Zur Herstellung einer isotonischen Suspension kann nach Beendigung der Ultraschallbehandlung Natriumchlorid in der erforderlichen Menge zugesetzt werden.

Beispiel 2

200 mg Gesamtlipid pro ml Flüssigkeit

Hergestellt aus 16 g L-α-Dipalmitoyl-lecithin und 4 g Cholesterin analog Beispiel 1.

Beispiel 3

0,1 mg Gesamtlipid pro ml Flüssigkeit

Hergestellt aus 3,3 mg L-α-Dipalmitoyl-lecithin und 6,6 g Cholesterin analog Beispiel 1.

Beispiel 4

10 mg Gesamtlipid pro ml Flüssigkeit

Hergestellt aus 500 mg D,L-α-Dipalmitoyl-lecithin und 500 mg Cholesterin analog Beispiel 1.

## Beispiel 5

### 0,013 mg Gesamtlipid pro ml Flüssigkeit

Hergestellt aus 1,2 mg L-α-Dipalmitoyl-lecithin und 0,1 mg Cholesterin analog Beispiel 1.

Die so erhaltenen Lipidpartikel-Suspensionen in einer Konzentration von 0,001 bis 20 g Lipid-Micellen pro 100 ml müssen, da bei den üblicherweise zur Anwendung kommenden Inhalationsgeräten nur eine Inhalation von maximal ca. 3 ml Flüssigkeit für den Patienten zumutbar ist, gegebenenfalls vor Anwendung aufkonzentriert werden. Diese Aufkonzentrierung muß ohne Veränderung der Lipid-Micellen erfolgen. Dies kann beispielsweise durch Ultrafiltration durch Ultrafiltrationsmembranen mit einer Ausschlußgrenze von 30 000 Daltons (z.B. Diaflo-Membranen YM 30, Firma Amicon) erfolgen, wobei es möglich ist, die Lipid-Micell-Dispersion im Verhältnis 1:10 aufzukonzentrieren.
Die derart vorbereitete Dispersion wird mit Hilfe handelsüblicher Inhalationsgeräte wie z.B. Inhalette, Firma Dräger, inhaliert. Eine für die Inhalation zu konzentrierte Dispersion kann mit Wasser bzw. physiologischer Kochsalzlösung verdünnt werden.

Zur Steigerung der Haltbarkeit kann es von Vorteil sein, die Lipid-Micell-Dispersion nach bekannten Verfahren zu lyophilisieren. Das gefriergetrocknete Produkt ist durch Wasserzugabe entsprechend der gewünschten Dosierung vor Gebrauch zu resuspendieren.

Desweiteren ist eine bekannte Variante der Inhalation das Einatmen von Pulvern mittels speziell hierfür auf dem Markt befindlichen Pulver-Inhalationsgeräten.

L-131

Ein getrocknetes Präparat ist somit auch, gegebenenfalls mit hierfür bekanntermaßen geeigneten Hilfsstoffen wie Milchzucker vermischt, geeignet zur trockenen Inhalation.

Die antibakterielle Wirksamkeit einer erfindungsgemäß hergestellten Surfactant-Mischung, nämlich einer Mischung bestehend aus Lecithin und Cholesterin in einem Gewichtsverhältnis von 4:1 wurde wie folgt geprüft:

a) Der Titer der lebenden Zellen, z.B. von Epithelzellen wie ATCC, CCL5, L-132, embryonic lung, human oder Hela markers, wird mittels Vitalfärbung mit Trypan blue und Auszählung in einer Fuchs-Rosenthal-Zählkammer bestimmt und durch Verdünnung mit Kulturmedium, z.B. Minimun Essential Medium plus 10 % fötales Kälberserum plus 0,1 % Kanamycin, auf $7 \times 10^4$ Zellen/ml eingestellt. Je 3 ml dieser Suspension werden in Glasszintillationsfläschchen gefüllt, die durch eine übliche Behandlung für die Anheftung der Epithelzellen auf die Glasoberfläche vorbereitet wurden. Die Inkubation bis zur Ausbildung eines geschlossenen Monolayers erfolgt über 3 Tage bei 37°C und 3 % $CO_2$. Ein Monolayer entspricht $5 \times 10^6$ Zellen pro Szintillationsfläschchen.

b) Die Bakterien, d.h. Klebsiella pneumoniae Weingarten 14, werden in zwei Passagen von je 24 Stunden bei 37°C in Brain Heart Infusion Broth (BAJ) bis zur logarithmischen Wachstumsphase gezüchtet. Während der 2. Passage erfolgt die radioaktive Markierung durch die Zugabe von $1,2-^{14}$-C-Azetat zum Nährmedium. Danach wird die Kultur zur Entfernung nichtinkorporierter Radioaktivität zweimal mit PBS (Phosphate buffered saline) gewaschen. Mit PBS pH 5,5 wird die Kultur im Photometer auf eine Transmission von 35 % eingestellt, was durch Ermittlung der Lebendkeimzahl einem Titer von $5 \times 10^8$ Bakterien/ml entspricht. Die Kultur wird abzentrifugiert, PBS abgenommen und durch ein gleiches Volumen Lipidsuspension ersetzt. Für den Kontrollwert wird statt der Lipidsuspension PBS pH 5,5 verwendet.

L-13¹

c) Das Nährmedium der Zellen wird abgenommen, der Monolayer (= 5 x $10^6$ Zellen) wird zweimal mit PBS pH 5,5 gespült und mit 1 ml Bakterien-Lipidsuspension (= 5 x $18^8$ Bakterien) überschichtet. Die Inkubation erfolgt 1,5 - 3 Stunden bei 37°C. Danach wird die Bakterien-Lipidsuspension abgenommen und der Monolayer zur Entfernung nicht-adhärenter Bakterien zweimal mit PBS pH 5,5 gespült.

Nach Zugabe von Instagel wird die Radioaktivität der gebundenen Bakterien im Szintillationszähler gemessen. Die minimale antiadhäsive Konzentration (MAK), die antiadhäsive Konzentration 50 % ($AK_{50}$) und die antiadhäsive Konzentration 90 % ($AK_{90}$) sind in der nachfolgenden Tabelle zusammengestellt.

MAK         200 ng x $ml^{-1}$

$AK_{50}$         6 mg x $ml^{-1}$

$AK_{90}$         30 mg x $ml^{-1}$

Das erfindungsgemäß hergestellte pharmazeutische Erzeugnis hemmt die Interaktion von Mikroorganismen mit Zellkulturen, wie z.B. von Erregern von Infektionen des Respirationstraktes mit Lungenepithelzellen, indem es durch eine Surfactantschicht verhindert, daß die Adhäsion der Mikroorganismen mit den Adhäsivrezeptoren der Zellen in Kontakt kommen.

Als derartige Erreger kommen beispielsweise Bakterien wie Actinomyces, Bacteroides, Bortedella pertussis, Corynebacterium diphteriae, Haemiphilus influenzae, Klebsiella, Mycobacterium tuberculosis, Mycoplasma pneumoniae, Nocardia, Peptokokken, Peptostreptokokken, Pneumokokken, Proteus mirabilis, Pseudomonas aeruginosa, Serratia, Staphylokokken, Streptokokken und Pilze wie Aspergillus und Candida in Betracht. Bereits adhärente wachsende Zellen werden wieder abgelöst.

Das Präparat gemäß der Erfindung eignet sich somit zur Behandlung und zur Prophylaxe von Infektionen wie solchen des Respirationstraktes (Nasen-Rachenraum einschließlich Nebenhöhlen), der Haut, der Pleura, des Peritoneums, des Urogenitaltraktes, der Gelenke, der Conjunktiven.

Beispielsweise kann eine bakterielle Zystitis durch Instillation einer erfindungsgemäßen Surfactant-Mischung in einer Konzentration von 8 mg Lipidpartikeln (4:1) je ml in die Harnblase mit Erfolg behandelt werden. Die Einzeldosis liegt hierbei zweckmäßigerweise bei 50 bis 150 mg, vorzugsweise jedoch bei 100 mg.

Außerdem haben klinische Versuche an Patienten mit Atemwegserkrankungen gezeigt, daß die Lipidsuspensionen gemäß der Erfindung eine hervorragende entzündungshemmende und damit antitussive und auch antiobstruktive Wirkung haben, welche der Wirkung der bisher angewendeten Präparate überlegen ist. Da es sich bei Lecithin und Cholesterin um körpereigene Substanzen handelt, die in geringer Dosierung zugeführt werden, kann das Auftreten toxischer Nebenwirkungen ausgeschlossen werden.

Aus der wässrigen Suspension kann durch Trocknung, insbesondere Gefriertrocknung, eine Trockensubstanz hergestellt werden, die zwecks Behandlung von Erkrankungen, z.B. der Atemwege, in pulverisierter Form in die Atemwege eingeführt werden kann.

Zur pharmazeutischen Anwendung lassen sich die Surfactant-Mischungen gemäß der Erfindung in die üblichen galenischen Zubereitungen, wie Pulver, Sprays oder Suspensionen zur Inhaltation, Injektion oder Instillation, einarbeiten.

Die Lipidsuspensionen gemäß der Erfindung ermöglichen eine ganz neue Behandlungsweise bei der Bekämpfung von Infektionen, Husten und obstruktiven Atemwegserkrankungen: die abzuschirmenden bzw. befallenen Körperzellstrukturen werden durch eine Surfactant-Schicht von den Mikroorganismen isoliert, sodaß deren Toxine die Zellen nicht mehr erreichen können und der Vermehrungsstoffwechsel der Mikroorganismen wegen der gehemmten Interaktion von Mikroorganismen und Zellen gestört ist.

Die Lipidsuspensionen gemäß der Erfindung wirken unspezifisch, d.h. sie haben ein sehr breites Wirkungspektrum, sie sind atoxisch (die Lipide verschwinden im Stoffwechsel) und haben demzufolge nicht die schädlichen Nebenwirkungen der bislang angewendeten Medikamente. Auch sind deren Gegenindikationen für die Präparate gemäß der Erfindung nicht gegeben, insbesondere ist die Gravidität keine Gegenindikation. Es sind auch keine Resistenzentwicklungen der Mikroorganismen zu erwarten.

Ansprüche:

1. Pharmazeutisches Erzeugnis zur Behandlung und Prophylaxe von Infektionen sowie von Husten und obstruktiven Atemwegserkrankungen, enthaltend Lecithin- und/oder Cholesterin-Partikel.

2. Pharmazeutisches Erzeugnis nach Anspruch 1, enthaltend 2 bis 250 mg Lipidpartikel.

3. Pharmazeutisches Erzeugnis nach Anspruch 1 oder 2, gekennzeichnet durch ein im Bereich von 1:2 bis 12:1 liegendes Lecithin-Cholesterin-Gewichtsverhältnis.

4. Pharmazeutisches Erzeugnis nach mindestens einem der vorhergehenden Ansprüche, dadurch gekennzeichnet, daß die Lipidpartikel in dreifach destilliertem Wasser suspendiert sind.

5. Pharmazeutisches Erzeugnis nach mindestens einem der Ansprüche 1 bis 3, dadurch gekennzeichnet, daß die Lipidpartikel in physiologischer Kochsalzlösung suspendiert sind.

6. Verfahren zur Herstellung eines pharmazeutischen Erzeugnisses nach mindestens einem der vorhergehenden Ansprüche 1 bis 5, dadurch gekennzeichnet, daß

a) Lecithin und/oder Cholesterin in der durch die gewünschte Zusammensetzung vorgegebenen Gewichtsmenge in einem mit Wasser nicht mischbaren geeigneten Lösungsmittel wie Chloroform gelöst werden, die so entstandene Lipidlösung mit Wasser überschichtet und anschließend kontinuierlich gemischt wird bis zu ihrer Umwandlung in flüssigen Schaum,

L-13'

der dann bis zur vollständigen Verdunstung des Lösungsmittels mit Ultraschall behandelt wird und gewünschtenfalls die so erhaltene wässrige Lipidpartikelsuspension einem Trocknungsvorgang unterworfen wird.

7. Verfahren nach Anspruch 6, dadurch gekennzeichnet, daß die Beschallung mit einer Leistung von ca. 90 bis 100 Watt erfolgt.

8. Verfahren nach Anspruch 6 oder 7, gekennzeichnet durch eine im Bereich von ca. 30 Minuten bis zu 5 Stunden liegende Beschallungszeit.

9. Verfahren nach mindestens einem der vorhergehenden Ansprüche 6 bis 8, dadurch gekennzeichnet, daß zum Überschichten der Lipidlösung in Chloroform dreifach destilliertes Wasser verwendet wird.

10. Verfahren nach mindestens einem der vorhergehenden Ansprüche 6 bis 9, dadurch gekennzeichnet, daß durch Zusatz von NaCl zur fertigen Suspension eine Isotonie hergestellt wird.

11. Verfahren nach mindestens einem der vorhergehenden Ansprüche 6 bis 10, dadurch gekennzeichnet, daß der Lipidlösung in Chloroform eine solche Menge an Wasser überschichtet wird, daß eine Lipidpartikel-Konzentration von 0,001 mg bis 0,2 gr je ml Wasser entsteht.

12. Verfahren nach mindestens einem der Ansprüche 6 bis 10, dadurch gekennzeichnet, daß die wässrige Lipidpartikelsuspension einer Gefriertrocknung unterworfen wird.